(19) Europäisches Patentamt / European Patent Office / Office européen des brevets

(11) **EP 2 155 368 B1**

(12) **EUROPÄISCHE PATENTSCHRIFT**

(45) Veröffentlichungstag und Bekanntmachung des Hinweises auf die Patenterteilung:
**05.06.2019   Patentblatt 2019/23**

(21) Anmeldenummer: **08749030.6**

(22) Anmeldetag: **21.04.2008**

(51) Int Cl.:
**B01D 69/08** *(2006.01)*     **B01D 61/02** *(2006.01)*
**B01D 61/14** *(2006.01)*     **B01D 69/02** *(2006.01)*
**B01D 69/12** *(2006.01)*     **B01D 71/44** *(2006.01)*
**B01D 71/68** *(2006.01)*     **A61M 1/16** *(2006.01)*

(86) Internationale Anmeldenummer:
**PCT/EP2008/003195**

(87) Internationale Veröffentlichungsnummer:
**WO 2008/128749 (30.10.2008 Gazette 2008/44)**

(54) **HOHLFASERKAPILLARMEMBRAN UND VERFAHREN ZU DEREN HERSTELLUNG**

HOLLOW FIBER CAPILLARY MEMBRANE AND METHOD FOR THE PRODUCTION THEREOF

MEMBRANE CAPILLAIRE A FIBRES CREUSES ET PROCEDE DE FABRICATION ASSOCIE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **23.04.2007   DE 102007129051**

(43) Veröffentlichungstag der Anmeldung:
**24.02.2010   Patentblatt 2010/08**

(73) Patentinhaber: **Fresenius Medical Care Deutschland GmbH**
**61352 Bad Homburg (DE)**

(72) Erfinder:
• **FISLAGE, Rainer**
**66606 St. Wendel (DE)**
• **HEILMANN, Klaus**
**66606 St. Wendel (DE)**
• **KELLER, Torsten**
**54411 Hermeskeil (DE)**
• **LICHAU, Holger**
**35325 Mücke/Ober-Ohmen (DE)**
• **RAIKO, Igor**
**66606 St. Wendel (DE)**
• **SANDER, Roland**
**66606 St. Wendel (DE)**

(74) Vertreter: **Stolmár & Partner Patentanwälte PartG mbB Blumenstraße 17 80331 München (DE)**

(56) Entgegenhaltungen:
JP-A- 2001 038 171     US-A- 5 141 642
US-A- 5 145 583     US-A- 5 298 206
US-A- 5 472 607     US-A- 5 863 645
US-A1- 2003 232 184     US-A1- 2006 108 288
US-B1- 7 172 075

## Beschreibung

Die vorliegende Erfindung betrifft eine Hohlfaserkapillarmembran und ein Verfahren zu deren Herstellung sowie deren Verwendung insbesondere in der Plasmapherese.

Kapillarmembranen unterschiedlicher Zusammensetzungen sind insbesondere aufgrund ihrer zunehmenden Verwendung in der Dialysetechnik oder auch in der Plasmapherese bekannt. Die Verwendung und die Herstellung von Membranen, insbesondere von Kapillarmembranen in der Dialysetechnik ist beispielsweise in der Veröffentlichung von Samtleben und Lysaght in: Hörl et al. Replacement of Renal Function by Dialysis 5th ed., Kluwer, 2004, S. 709 bis 724 beschrieben.

So beschreibt die WO 96/37282 eine Membran insbesondere für die Hämodialyse die eine Trennschicht mit einem cut-off zwischen 500 und 500000 Dalton, eine Stützschicht und eine die hydraulische Permeabilität mitbestimmende Schicht aufweist, wobei die Trenngrenze und hydraulische Permeabilität unabhängig voneinander eingestellt werden. Der Aufbau der Membran mit unterschiedlichen Porengrößen innerhalb der einzelnen Schichten ist jedoch sehr aufwendig.

Die EP 1547628 A1 beschreibt Plasmareinigungsmembranen und ein System zur Reinigung von Plasma, und stellt insbesondere auf spezifische physikalische Eigenschaften auf die Bruchfestigkeit der Membran aufgrund der starken Beanspruchung bei der Plasmareinigung ab. Hierbei kommt es insbesondere auf die Protein- und die Immunoglobulinpermeabilität an. Dabei wird in der Membran mit einer Schwammstruktur ein Gradient der Porengröße eingestellt, wobei an der äußeren Oberfläche eine größere Porengröße zu finden ist als an der inneren Oberfläche der Membran.

Das US 6,565,782 betrifft synthetische polymere Mikrofiltrationmembranmaterialien mit hoher Oberflächenporosität erhältlich durch Co-Fällen eines Sulfonpolymers mit einem hydrophilen Polymer wie Polyvinylpyrrolidon. Nachteile treten bei dieser Membran insbesondere in Bezug auf die Trennung zellulärer Bestandteile des Blutes von der Plasmaphase auf, da der Druck auf die Blutzellen bedingt durch den Einsatz kleinporiger Membranen zur Schädigung der Blutzellen führen kann.

Die US 5 141 642 A offenbart eine Hohlfasermembran, deren Wand aus einem doppellagigen aromatischen Polyimid aufgebaut ist und die eine hervorragende Hitzebeständigkeit, chemische Widerstandsfähigkeit und eine Druckbeständigkeit aufweist.

Die innere Schicht weist eine Dicke von 10 bis 500 $\mu$m auf und ist mikroporös und besteht aus einem ersten aromatischen Imidpolymer, das in organischen Lösungsmitteln löslich ist und die äußere Schicht besteht im Wesentlichen aus einem zweiten aromatischen Imidpolymer, das ebenfalls in organischen Lösungsmitteln löslich ist und eine Dicke von 2 bis 200 $\mu$m aufweist.

Die US 5 472 607 A offenbart eine Hohlfasermembran, die einen röhrenartigen makroporösen Träger umfasst, auf dessen Oberfläche ein dünner semipermeabler Kunststofffilm aufextrudiert ist, der durch Extrusion einer einzigen Lösung, die Polysulfon und Polyvinylpyrrolidon enthält, erhalten werden kann.

Die US 2003/232184 A1 offenbart eine poröse Membran, die ein dreidimensionales Netzwerk und eine sphärische Struktur aufweist, wobei der durchschnittliche Durchmesser der sphärischen Struktur im Bereich von 0,1 bis 10 $\mu$m liegt. Die durchschnittliche Porengröße des dreidimensionalen Netzwerks liegt im Bereich von 5 nm bis 50 $\mu$m. Die Oberfläche der porösen Membran weist eine durchschnittliche Porengröße von 0,005 bis 0,5 $\mu$m auf.

Die US 5 145 583 A betrifft eine asymmetrische semipermeable Membran, die mindestens ein hydrophobes Polymer und mindestens ein Copolymer basierend auf Acrylonitril, mindestens ein sulfonisches Monomer und optional ein nichtionisches, ionisierbares, olefinisches ungesättigtes Monomer umfasst, und die mittels Koextrusion hergestellt wird.

Die US 2006/108288 A1 offenbart eine Plasmareinigungsmembran in Form einer Hohlfaser, die aus einem hydrophoben Polymer und einem hydrophilen Polymer erzeugt wurde und eine schwammartige Struktur aufweist, und die als Bestandteile aromatisches Polysulfon und Polyvinylpyrrolidon enthalten kann. Die Herstellung der Membran erfolgt aus einer einzigen Spinnlösung.

Weitere Membranen sind im Stand der Technik aus den Dokumenten US 5 863645 A, US 7 172 075 B1 und US 5 298 206 A bekannt. JP2001038171 A offenbart eine Hohlfaser-Blutdialysemembran aus Polyestersulfon und Polyvinylpyrrolidon.

Zur Herstellung derartiger Kapillarmembranen werden zumeist so genannte Hohlfaserdüsen verwendet. Eine Übersicht über diese und weitere Techniken zur Herstellung von Hohlfasermembranen sind in M. Mulder, Basic Principles of Membrane Technology second ed., Kluwer 1996, S. 71-91 offenbart.

Bei der Herstellung einer Hohlfasermembran mittels einer Hohlfaserdüse wird die Hohlfasermembran in einem so genannten Fällungsspinnprozess hergestellt, wobei die auszufällenden Polymere aus einem Ringspalt einer Düsenanordnung austreten, während das entsprechende Fällungsmittel aus einer zentralen Fällungsmittelbohrung ausströmt.

Eine Hohlfaserdüse der genannten Art ist beispielsweise in der DE 10211051 A1 offenbart.

Typische Plasmapheresefilter des Standes der Technik enthalten zumeist hydrophobe Membranen beispielsweise aus Polypropylen, Polysulfon etc.

Da diese hydrophoben Membranen nicht mit Wasser benetzbar sind, werden die diese Membranen enthaltenden Filter typischerweise mit Wasser unter Druck hydrophilisiert. Für die nachfolgende Blutbehandlung ist somit sichergestellt, dass alle Lufteinschlüsse innerhalb der Poren ausgetrieben worden sind und somit nicht in den Blutkreislauf gelangen. Nachteiligerweise müssen diese Filtermodule mit hydrophoben Hohlfasermembranen mit Wasser befüllt an den Kunden und den Patienten ausgeliefert werden. Die Rohstoff- und Vertriebskosten und die Schwierigkeit, die Sterilität solcher befülltem Module zu gewährleisten sind Anforderungen, die man bestrebt ist zu umgehen.

Probleme bei den bisher bekannten Plasmamembranen sind deren geringe Permeabilität für große Lipoproteine sowie druckinduzierte Schädigungen von Blutzellen durch die transmembrane Druckdifferenz, d.h. durch die negativen Drücke, die auf eine an der Membranwand anhaftenden und an einer Porenöffnung anliegenden Blutzelle einwirken. Je geringer die Porengröße, desto höher ist bei gegebener transmembraner Druckdifferenz (TMP), die auf eine relativ zur Porengröße große Blutzelle einwirkende Druckdifferenz, auf den betroffenen Abschnitt der Blutzelle. In solchen Fällen hat sich oftmals gezeigt, dass der auf den entsprechenden Abschnitt der Blutzelle einwirkende Druck so groß ist, dass die Blutzellwände zerplatzen und es zur Hämolyse kommt. Man ist daher bestrebt, an der blutseitigen Membranwandfläche eine möglichst hohe Porosität herzustellen, so dass sich die negative Druckeinwirkung auf die Blutzellwand über einen größeren Flächenbereich der Blutzelle verteilt.

Aufgrund der geringen Permeabilität der aus dem Stand der Technik bekannten Membranen für große Liproproteine treten bei diesen insbesondere Schwierigkeiten bei der Filtration von lipämischem Blut durch den Abfall der Siebkoeffizienten auf. Hydrophobe Plasmamembranen zeigen in der Blutbehandlung oftmals die negative Eigenschaft, sich im Laufe der Behandlung durch Wechselwirkung mit den unpolaren Blutfetten zuzusetzen. Man beobachtet daher oftmals einen Abfall des Siebkoeffizienten während des Verlaufs der Blutbehandlung.

Aufgabe der vorliegenden Erfindung war es daher, eine Hohlfasermembran bereitzustellen, die insbesondere eine schonende Plasmapherese, insbesondere eine schonende Plasmafiltration von Blut ermöglicht. Weiter sollte eine derartige Hohlfasermembran neben möglichst großen Öffnungen für eine gute Lipoproteinpermeabilität bei gleichzeitig hoher Selektivität auch eine hohe Porosität für eine verbesserte Blutkompatibilität aufweist.

Die erfindungsgemäße Aufgabe wird gelöst durch den Gegenstand der unabhängigen Ansprüche. Erfindungsgemäß wird diese Aufgabe durch eine integrale Hohlfasermembran gelöst, bestehend aus zwei koextrudierten Schichten A und B, wobei Schicht B eine vliesartige Struktur mit einer Maschenweite von 0,1 bis 10 μm aufweist und Schicht A eine Porenstruktur aufweist. Unter dem Begriff "Maschenweite" ist in diesem Zusammenhang in einer vlies- oder netzartigen Struktur der weiteste Abstand zwischen den einzelnen Verzweigungen der das Vlies oder Netz bildenden Struktur zu verstehen. Die Dicke der Stege der Verzweigung beträgt dabei 0,1 - 0,5 μm.

Bevorzugt bildet dabei die Schicht B die so genannte Blutkontaktseite und Schicht A die Filtratseite der Hohlfasermembran beispielsweise bei einer Blutbehandlung, bei der das Blut durch das Hohlfaserinnere geleitet wird.

Im Regelfall ist die Blutkontaktseite die Innenschicht der Hohlfasermembran und die Schicht A, also die Filtratseite die Außenschicht der Membran. In weniger bevorzugten Ausführungsformen ist es jedoch auch möglich, dass Schicht B die Außenschicht (die Blutkontaktseite) und Schicht A die Innenschicht (Filtratseite) ist.

Durch die erfindungsgemäße Membran und insbesondere durch das Vorhandensein der vliesartigen Innenschicht B wirkt auf einen Abschnitt einer Blutzelle durch die transmembrane Druckdifferenz ein geringerer negativer Druck ein, als bei einer kleinporösen Membran des Standes der Technik, so dass insbesondere die zellulären Bestandteile des Bluts besonders schonend von der Plasmaphase des Bluts getrennt werden können.

Bevorzugt ist, dass Schicht A aus mindestens drei aufeinanderfolgende Zonen A1, A2, A3 unterschiedlicher Porosität besteht, wobei Zone A1 die Oberfläche der Schicht A bildet und Poren mit einer mittleren Porengröße von 0,7 bis 2 μm aufweist. Die Dicke der Zone A1 liegt typischerweise im Bereich von 9 bis 11 μm, bevorzugt bei 10 μm bei einer bevorzugten Wandstärke von ca. 60 μm.

Daran anschließend findet sich die Zone A2, die zwischen den Zonen A1 und A3 angeordnet ist und eine mittlere Porengröße von größer als 200 nm aufweist.

Die Dicke dieser Zone A2 beträgt typischerweise ca. 10 μm bei einer bevorzugten gesamten Wandstärke von 60 μm. Generell beträgt die Dicke der Zone A2 also etwa 1/6 der Gesamtwandstärke.

Eine dritte Zone A3 ist zur Schicht B direkt benachbart und ist typischerweise in schlüssiger Verbindung mit der vliesartigen Struktur der Schicht B. Die Zone A3 weist einen Porengrößegradienten zur Schicht B hin auf, d.h. die Porengröße nimmt zur Schicht B hin zu. Die Dicke der Zone A3 beträgt ungefähr 30 μm bei einer gesamten Wandstärke von 60 μm. Generell beträgt die Dicke der Zone A3 also ca. 50 % der Gesamtwandstärke.

Die Schichtdicken der Zonen A1, A2, A3 sind in Relation zur Gesamtwandstärke ausgebildet. Eine Vergrößerung der Gesamtwandstärke um z. B. 100 % wird auch die Schichtdicke der einzelnen Zonen um ca. 100 % vergrößern, wobei die Relationen der Schichtdicken untereinander konstant bleiben. Beim Übergang zu noch größeren Schichtdicken der Gesamtwandstärke wurde allerdings bei der Herstellung festgestellt, dass sich die Relationen der Schichten zueinander verändern, insbesondere die Schichtdicke der Schicht A2 relativ weniger stark ausfällt, als bei dünnwandigeren Membranen.

Wichtig bei der erfindungsgemäße Kapillarmembran, die aus zwei koextrudierten Schichten A und B besteht, ist, wie schon vorstehend gesagt, die unterschiedliche Porengröße bzw. Maschengröße in den Schichten A und B, wobei die Maschengröße der Maschen in Schicht B nicht nur in Bezug auf die Porengröße der vorstehend erwähnten äußersten Zone der Schicht A der Zone A1 größer ist, sondern auch in Bezug auf sämtliche Poren der gesamten Schicht A.

Die beiden Schichten A und B erfüllen erfindungsgemäß unterschiedliche Funktionen:

Die äußere Schicht A verleiht der erfindungsgemäßen Hohlfasermembran durch ihre größere Massendichte ihre mechanische Stabilität, insbesondere auch bei der Herstellung der Membran gemäß dem erfindungsgemäßen Verfahren, das weiter unten im Detail beschrieben ist. Weiterhin weist diese Schicht in der gesamten Membran die Zone mit dem geringsten mittleren Porendurchmesser auf (größer als 200 nm) und ist somit im Hinblick auf die Filtration die selektionsbestimmende Schicht. Die Funktion der Schicht A besteht somit in der Verleihung von Stabilität und Selektivität der erfindungsgemäßen Membran.

Die bevorzugt innen angeordnete Schicht B, d.h. die dem Blut oder einer anderen Körperflüssigkeit zugewandte Schicht weist in ihrer netzartigen Struktur eine Maschenweite auf, die viel größer ist als die Porenweite der Schicht A. Nicht zuletzt durch ihre netz- oder vliesartige Struktur und der daraus resultierenden geringen Massendichte, weist diese Schicht fast keine mechanische Festigkeit auf und muss daher durch die zusätzliche Schicht A gestützt werden. Die Schicht B hat die Aufgabe, im Blutbehandlungsverfahren lediglich die zellulären Bestandteile der hindurchzuleitenden Flüssigkeit zurückzuhalten.

Durch den vliesartigen Aufbau dieser Schicht hat sich überraschenderweise ergeben, dass dies gegenüber den Zellen auf unerwartet schonende Weise vonstatten geht. Diese Schicht hat somit im Wesentlichen die Funktion der Kompatibilisierung zwischen der zu filtrierenden Flüssigkeit und der Membran.

Der vliesartige Aufbau und die damit verbundene hohe Porosität der Schicht B ergeben weiter überraschenderweise einen verbesserten und über den Behandlungsverlauf konstanten Siebkoeffizienten für hochmolekulare Bestandteile des Blutes wie z. B. Triglyceride oder Lipoproteine. Es hat sich dabei gezeigt, dass die Siebkoeffizienten über einen längeren Behandlungsverlauf, im Gegensatz zu bisher bekannten Plasmamembranen, im Wesentlichen konstant bleiben. So stellt man bei bisher bekannten Plasmamembranen fest, dass die Poren der inneren Oberfläche durch große im Blut vorhandene Blutfettpartikel zugesetzt werden können. Als Folge beobachtet man, dass die Siebkoeffizienten absinken, da eine geringere gesamte Durchflusspassage durch die Membranwand zur Verfügung steht und die effektive Permeabilität absinkt. Die Porosität der erfindungsgemäßen Membran ist dagegen an der Blutkontaktseite so groß, dass selbst durch Adsorption der großen Blutfettpartikel genügend Fluidpassagen zur Verfügung stehen, um die gewünschte Permeabilität aufrecht zu erhalten.

Um der Membran die optimalen Eigenschaften bezüglich Stabilität und Selektivität zu verleihen, beträgt das Verhältnis der Schichtdicken von Schicht A zu Schicht B 4:1 bis 6:1, so dass Schicht A insbesondere ihre stützende Stabilitätsfunktion besonders gut erfüllen kann.

Es hat sich gezeigt, dass ein Innendurchmesser von 280 bis 400 $\mu$m für den geplanten Einsatz von Vorteil ist, um auch stärkere Drücke und Druckdifferenzen auszuhalten. Typische Gesamtwandstärken der erfindungsgemäßen Hohlfasermembranen liegen bei 40 bis 80 $\mu$m, ganz besonders bevorzugt von 60 $\mu$m. Derartige erfindungsgemäße Membranen werden typischerweise in Faserbündelgrößen von 1300 bis 2600 Fasern zur Herstellung von Plasmafiltern mit 0,3 und 0,6 m$^2$ Membranfläche verwendet.

Die Membranfläche bedingt auch die physikalischen Parameter der Membran: Ein Plasmafilter mit einem Bündel aus einer Vielzahl erfindungsgemäßer Hohlfasermembranen ("Hohlfaserbündel") mit einer Gesamt-Membranfläche von 0,3 m$^2$ ist für den Einsatz bei Blutflüssen von 100 ml je Minute und Filtratflüssen von bis zu 30 ml/m vorgesehen, der Plasmafilter mit 0,6 m$^2$ Membranfläche für Blutflüsse von 200 ml je Minute und Filtratflüsse bis zu 3 ml/min.

Jede Lage besteht aus einem Polymergemisch von mindestens zwei Polymeren.

Erfindungsgemäß ist das Material der Schichten A und B eine Mischung aus Polysulfon und Polyvinylpyrrolidon.

Die Konzentration beider Komponenten in den unterschiedlichen Lagen können entsprechend der Anforderung an die Membranstruktur unabhängig voneinander eingestellt werden. Eine hohe Polymerkonzentration für die äußere Lage ergibt hohe Viskositäten in der noch nicht ausgefällten Membran und damit insbesondere eine geringe Porosität und eine geringe Polymerkonzentration für die innere Schicht B ergibt hochporöse vliesartige Membranstrukturen.

Die Aufgabe der vorliegenden Erfindung wird weiter durch ein Verfahren zur Herstellung einer erfindungsgemäßen Hohlfasermembran gelöst, umfassend die Schritte des

(a) Bereitstellens zweier Spinnmasselösungen A und B, wobei die Viskosität der Spinnmasselösung A höher als die Viskosität der Spinnmasselösung B,
(b) Einstellens der Fällbadtemperatur auf größer 70°C,
(c) das in Kontaktbringen der beiden Spinnmasselösungen A und B durch eine Hohlfaserdüse mit einem inneren Fällungsmittel,
(d) Fällens der Hohlfasermembran,

wobei die Spinngeschwindigkeit 200 bis 400 mm/s beträgt, dadurch gekennzeichnet, dass

die Viskosität der Spinnmasselösung A im Bereich von 8000 bis 15000 mPa•s liegt,

wobei die Viskosität der Spinnmasselösung B weniger als 1000 mPa•s beträgt, und

wobei die Fällspalthöhe 5 bis 50 mm beträgt.

Die Einstellung der Fälltemperatur von mehr als 70°C, insbesondere mehr als 75°C ermöglicht eine höhere Feuchtigkeit in der Umgebung des Fällspaltes, so dass sich an der Außenseite der Membran Poren mit einem geringen Durchmesser, insbesondere in der erfindungsgemäß beschriebenen äußersten Lage bilden.

Je nach Anteil der einzelnen Bestandteile wird damit auch die Spinnmassenviskosität eingestellt. Diese ist vom Molekulargewicht der Einzelkomponenten abhängig.

Die Viskosität der Spinnmasselösung A beträgt erfindungsgemäß 8000 bis 15000 mPa•s, insbesondere 9000 bis 14000 mPa•s, je nach erwünschter Membranstruktur. Dabei enthält die Spinnmasselösung A typischerweise 15 bis 25 % Polysulfon (PSU), 4 bis 8 % Polyvinylpyrrolidon (PVP) und 81-67 % Lösungsmittel (98-100 % DMAC und 0-2 % Wasser). Bevorzugt sind 17,5-22,5 % PSU, 5-8 % PVP, Rest Lösungsmittel (80-100 % DMAC und 0-20 % Wasser). Ganz besonders bevorzugt sind 19-21% PSU, 5,5-7 % PVP, Rest Lösungsmittel (98-100 % DMAC, 2-0 % Wasser). Die Prozentangaben beziehen sich soweit nicht anders angegeben stets auf Gew.%.

Die Bestimmung der Viskosität erfolgte mittels eines Rotationsviskosimeters (VT 550 der Fa. Haake), das auf 40° temperiert war mittels der nachstehenden Vorschrift:

Bei der Viskositätsmessung befand sich die Prüfsubstanz im Ringspalt zwischen konzentrisch angeordneten Zylindern, dem "Drehkörper" und "Messbecher". Die Drehzahl wurde vorgegeben und die dabei wirksame Kraft (Schubspannung) gemessen. Zunächst wurden das Temperiergefäß und der Drehkörper MV-DIN mit dem Grundgerüst verschraubt. Anschließend wurde der Nullpunkt überprüft bzw. eingestellt. Der Drehmotor wurde abgeschaltet und die Drehmoment-Anzeige mit der dafür vorgesehenen Taste auf Null eingestellt. Zur eigentlichen Messung wurde der Messbecher mit der Prüflösung luftblasenfrei bis zur entsprechenden Einfüllmarkierung gefüllt und mit der Arretierverschraubung im Temperiergefäß fixiert. Anschließend wurde die Drehzahlstufe vorgegeben. Das Programm wurde eingestellt und die Viskosität nach Ablauf der Messzeit abgelesen.

Für die Messung wurde an dem Gerät die Drehzahlstufe 3 gewählt. Die Messung dauerte 30 min. Der Viskositätswert wurde nach Vorgabe des im Gerät eingestellten Programms abgelesen. Für Messungen im manuellen Modus wurde das Programm S1 gewählt.

Die Viskosität der Spinnmasselösung B beträgt bevorzugt weniger als 1000 mPa·s und enthält 5 bis 15 % Polysulfon, 4 bis 8 % Polyvinylpyrrolidon und 91-77 % Lösungsmittel (100 % DMAC). Bevorzugt sind 7-13% PSU, 4-7 % PVP, Rest Lösungsmittel (100 % DMAC). Ganz besonders bevorzugt sind 8-12% PSU, 5-7 % PVP, Rest Lösungsmittel (100 % DMAC).

Die fertige Membran weist nach Spül- und Trocknungsschritten einen PVP-Gehalt von ca. 3% auf. Dieses PVP ist gebunden und nur minimal eluierbar.

Wichtig in diesem Zusammenhang ist, wie vorstehend gezeigt die unterschiedliche Viskosität der beiden Spinmasselösungen A und B, wodurch die unterschiedliche Porosität in den beiden koextrudierten Lagen A und B der erfindungsgemäßen Hohlfasermembran resultiert.

In Bezug auf die Viskosität der Spinnmasselösung B muss weiter darauf geachtet werden, dass die Viskosität nicht zu gering ist, typischerweise nicht weniger als 300 mPa·s, da ansonsten das Phänomen der so genannten Perligkeit auftritt, welches ein Vorstadium zum Abtropfen darstellt. Dabei fließt das Fällmittel nicht mehr gleichförmig, wodurch sich der Innendurchmesser in schneller Folge ändert, so dass der Hohlfaden wie eine Perlenkette erscheint. Dies tritt insbesondere dann auf, wenn die Spinnmassenlösung B eine Viskosität von weniger als 300, insbesondere weniger als 200 mPa·s aufweist und weich gefällt wird. In diesem Zusammenhang bedeutet der Begriff "weich gefällt", dass im Fällmittel des Fäll- bzw. Koagulationsbads ein hoher Lösungsmittelanteil vorhanden ist, woraus ein langsames Koagulieren des Polymerfadens resultiert und zu größeren Poren führt.

Im Rahmen der Erfindung kann die Membrandimension, d.h. auch die Wanddicke und der Innendurchmesser in verhältnismäßig weiten Bereichen variiert werden, wodurch es möglich ist, die Membran den verschiedenen Einsatzzwecken anzupassen. Für die Hämodialyse, Hämodiafiltration und Hämofiltration sowie bei der Plasmapherese beträgt die Wanddicke typischerweise 10 bis 70 $\mu$m und bei der Anwendung in der Ultrafiltration kann die Wanddicke einige 100 $\mu$m, z. B. 1000 $\mu$m betragen, wobei die Dimensionen nach oben und nach unten vom Fachmann angepasst werden können.

Bei Ausfällung mit einem Fällungsmittel, z. B. einer Mischung aus Dimethylacetamid (DMAC) und Wasser, beispielsweise 70 % DMAC und 30 % Wasser, bevorzugt 80 % DMAC und 20 % Wasser, entsteht mittels des erfindungsgemäßen Verfahrens die gewünschte erfindungsgemäße vliesartige, großporige Struktur der Schicht B.

Von besonderer Wichtigkeit ist auch die Geschwindigkeit der Fällung, was durch die Spinngeschwindigkeit von 200 bis 400 mm je Sekunde, ganz besonders bevorzugt 200 bis 250 mm je Sekunde eingestellt wird sowie durch eine Fällspalthöhe von 5 bis 50 mm.

Um die erforderlichen großen Poren in der Lage B zu erzeugen, muss die Spinnmasse langsam gefällt werden, so dass die entstehende Hohlfasermembran im Fällspalt sehr weich und mechanisch instabil bleibt.

Im Bereich der erfindungsgemäß eingestellten Spinngeschwindigkeit kann das weiche Fällmittel nicht die gesamte Membranwand durchwandern und die Membran tritt in das Fällbad (oder Koagulationsbad) ein, ohne dass sich an der Außenseite schon Poren gebildet haben. Die Bildung der Poren an der Außenseite wird, wie schon vorstehend ausgeführt, durch eine möglichst hohe Feuchtigkeit in der Umgebung des Fällspalts initiiert, die durch die Temperatur des Fällbades eingestellt wird. Nach dem Austritt aus der Extrusionsdüse wird der Polymerfaden bevorzugt in einer Einhausung (z. B. einem Rohr oder ähnlichem) bis zur Oberfläche des Fällbads geführt. In der Einhausung ist die Feuchtigkeit regulierbar.

Die erfindungsgemäß erhaltene Membran enthält noch große Mengen an herauslösbarem freien Polyvinylpyrrolidon in einer Menge von ca. 1 g/m$^2$. Dies wird in einem Spülbad mit einem Lösungsmittel, wie z.B. Wasser, herausgespült.

Die Temperatur des Spülbades wird dabei typischerweise im Bereich von 60 bis 80°C gehalten. Die Membran muss so weit wie möglich von Polyvinylpyrrolidon befreit werden, da ansonsten eluierbares PVP in den Blutkreislauf gelangen kann. Dies kann bevorzugterweise auch durch Trocknungstemperaturen der erfindungsgemäß erhaltenen Membran im Bereich von 80 bis 110, insbesondere von 90 bis 100°C vermieden werden.

Weitere Gegenstände der vorliegenden Erfindung sind die Verwendung der erfindungsgemäßen Hohlfasermembran für Trennprozesse im Nanofiltrations- und im Ultrafiltrationsbereich, insbesondere für die Hämodialyse und die Hämodiafiltration und die Hämofiltration.

Die erfindungsgemäßen Zweikomponentenmembranen verfügen über gute mechanische Eigenschaften wie Festigkeit, hohe Bruchdehnung im trockenen Zustand. Die Membranen können im Filtermodul trocken gelagert und trocken versandt werden. Von besonderer Bedeutung für die Blutbehandlungs-Anwendungsverfahren ist die Tatsache, dass mit der erfindungsgemäßen Hohlfaser ausgestattete Filtermodule ohne weiteres durch Blut benetzbar sind.

Die Erfindung wird anhand der Abbildungen und eines Ausführungsbeispiels näher erläutert, wobei diese jedoch nicht als einschränkend verstanden werden sollen.

Es zeigen

Abbildung 1: eine REM-Aufnahme der Schicht B in 1000-facher Vergrößerung,

Abbildung 2: eine REM-Aufnahme der Schicht B in 5000-facher Vergrößerung,

Abbildung 3: eine REM-Aufnahme der Schicht A einer erfindungsgemäßen Hohlfasermembran,

Abbildung 4: eine REM-Aufnahme der Querschnitts durch eine erfindungsgemäße Hohlfasermembran, und

Abbildung 5: eine REM-Aufnahme eines Längsschnitts durch eine erfindungsgemäße Hohlfasermembran.

Ausführungsbeispiel

Es wurde eine erfindungsgemäße Hohlfasermembran dargestellt, wobei die Spinnmassenlösung A aus 20 % Polysulfon (Fa. Solvay Udel P3500.LCD), 6 % Polyvinylpyrrolidon (ISP, PVP-K90) und 1 % Wasser und Rest Dimethylacetamid bestand und die Spinnmassenlösung B für die innere Lage B aus 10 Gew.-% Polysulfon, 5,5 % Polyvinylpyrrolidon Rest Dimethylacetamid bestand.

Das Fällungsmittel bestand zu 80 % aus Dimethylacetamid und zu 20 % aus Wasser.

Als Spinndüsen wurde eine Spinndüse gemäß der DE 10211051 verwendet, die in einem Spinnblock eingebaut war.

Die Spinnblocktemperatur wurde auf 60°C eingestellt. Die Fällspalthöhe betrug 30 mm und die Spinngeschwindigkeit 250 mm je Sekunde.

Die Temperatur des Fällbads betrug ca. 80°C.

Nach Fällung und Trocknung wurde die so erhaltene erfindungsgemäße Hohlfasermembran mittels REM Aufnahmen untersucht.

Die REM-Aufnahmen wurden mittels eines handelsüblichen Rasterelektronenmikroskops erstellt.

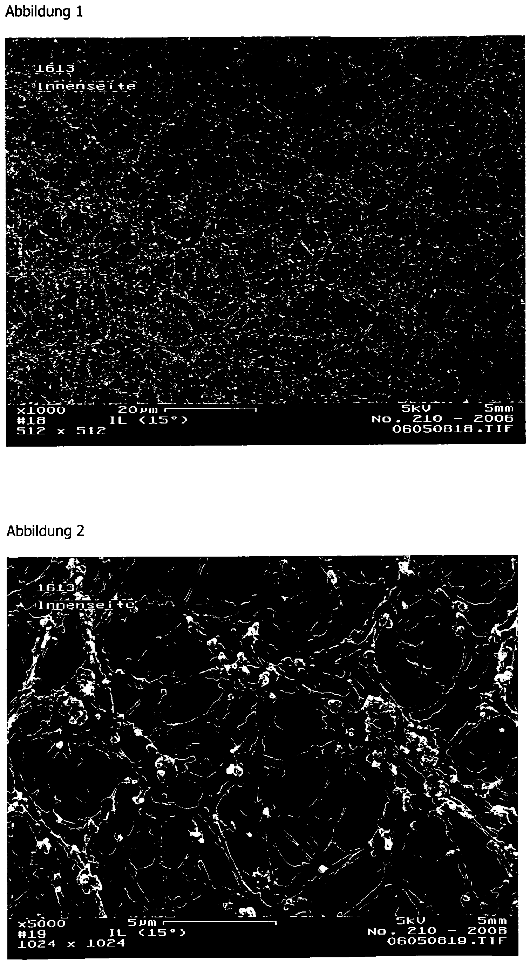

Abbildungen 1 und 2 zeigen REM-Aufnahmen in 1000-facher (Abbildung 1) sowie 5000-facher (Abbildung 2) Vergrößerung der Schicht B, dass heißt, der Innenseite der erfindungsgemäßen Hohlfasermembran.

Beide Aufnahmen zeigen die vliesartige Struktur der Schicht B, die aus vielen netzartigen Verstrebungen (Stege) aufgebaut ist. Diese vliesartige Struktur ist keine klassische Porenstruktur im herkömmlichen Sinne wie sie beispielsweise in der Schicht A vorliegt.

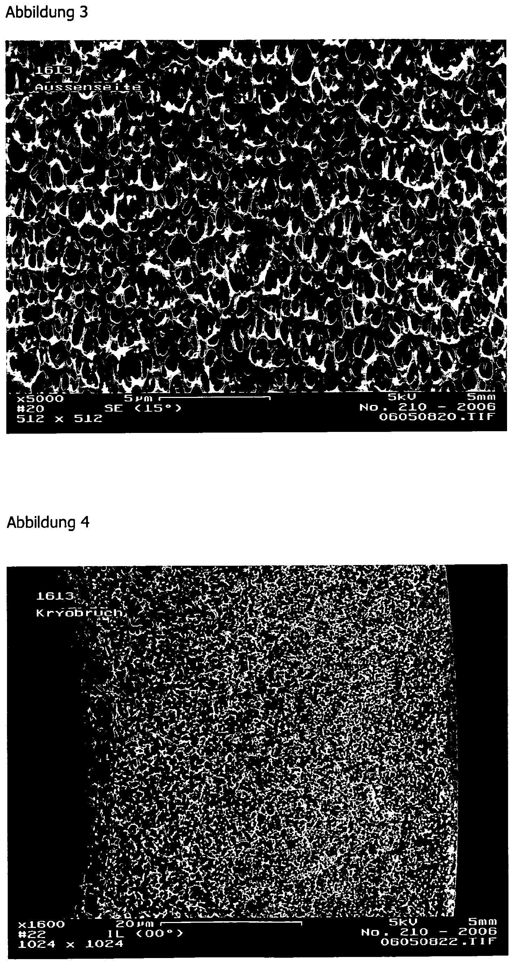

Abbildung 3 zeigt eine REM-Aufnahme in 5000-facher Vergrößerung der Außenseite der erfindungsgemäßen Hohlfasermembran (Schicht A) mit einer durchschnittlichen Porengröße von ca. 1 μm als Ergebnis des hohen Feuchtigkeitsgehalt im Fällspalt bei der Ausfällung. Insgesamt ist eine sehr hohe Porendichte mit einem geringen Anteil an Matrixmaterial erkennbar.

In Abbildung 4 ist eine REM-Aufnahme in 1600-facher Vergrößerung des Querschnitts durch eine erfindungsgemäße Hohlfasermembran gezeigt, der durch so genannten "Kryobruch" freigelegt wurde. Der Begriff "Kryobruch" bedeutet, dass die erfindungsgemäße Hohlfasermembran in flüssigen Stickstoff getaucht und anschließend von Hand in Querrichtung gebrochen wird.

Der zweilagige Aufbau der erfindungsgemäßen Membran ist aus Abbildung 4 ersichtlich, wobei bedingt durch den Zonenaufbau der Schicht A eine klare Grenzlinie zwischen den beiden Schichten A und B nicht sehr stark ausgeprägt ist, sondern beide über die erfindungsgemäß erhaltenen Gradienten in den einzelnen Zonen der Schicht A ineinander langsam übergehen.

Weiter wurden Messungen der Ultrafiltrationsrate, der Siebkoeffizienten und der Permeabilität durchgeführt.

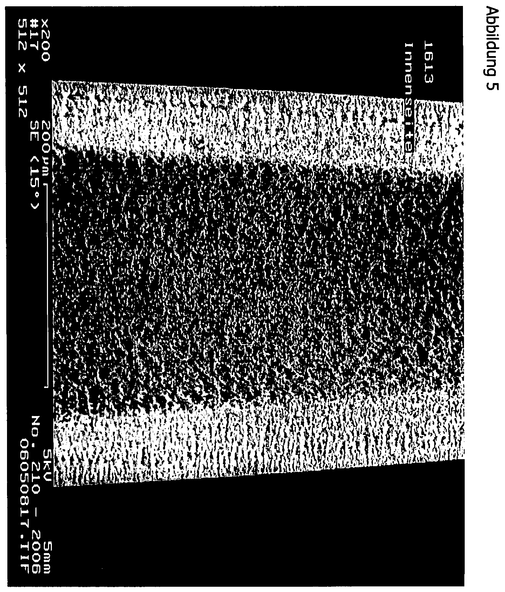

In Abbildung 5 ist eine REM-Aufnahme in 200-facher Vergrößerung des Längsschnitts durch eine erfindungsgemäße Hohlfasermembran gezeigt.

Der Längsschnitt wird erhalten, indem mit einer geeigneten Schneidvorrichtung, beispielsweise einem so genannten Mikrotommesser, die erfindungsgemäße Hohlfaser in Längsrichtung durchgeschnitten wird.

In der Abbildung stammen die unregelmäßigen Strukturen in der Hohlfasermembranwand von den Schnittspuren des Mikrotommessers.

In Abbildung 5 ist die vliesartige Netzstruktur der Innenseite der erfindungsgemäßen Hohlfasermembran gut zu erkennen.

*Ultrafiltrationsrate*

Die wässrige Ultrafiltrationsrate der erfindungsgemäßen Hohlfasermembran wurde gemäß der folgenden Gleichung

$$UF = V_{Filtrat} \text{ x } 3600)/t \text{ x } ((p_{ein} + p_{aus})/2) \text{ x } 0,75)$$

mittels eines aus dem Stand der Technik bekannten Dialyseschlauchsystems bestimmt, wobei UF die Ultrafiltrationsrate in (ml/ (h x mm Hg), $V_{Filtrat}$ das Filtratvolumen in ml (im vorliegenden Fall: 1000 ml), t, die Zeit in Sekunden (um 1000 ml zu filtrieren), $p_{ein}$ den Druck des blutseitigen Einlaufs (mbar) und $p_{aus}$ den Druck des blutseitigen Auslaufs (mbar) an der Vorrichtung bezeichnen.

Der Blutausgang (blutseitiger Auslauf) wurde während der Messung geschlossen, so dass nur Filtration erfolgte.

Für die erfindungsgemäßen Membranen (Oberfläche 0,6 m$^2$) wurde ein Ultrafiltrationswert (UF-Wert) im Bereich von 4500 bis 5000 ml/h x mmHg x m$^2$ gemessen.

*Siebkoeffizient*

Für ein Modul mit einer Oberfläche von 0,6 m$^2$ werden 1000 ml lipämisches Vollblut mit einen Triglyceridgehalt von 200-300 mg/dl eingesetzt. Dieses Blut wird für eine Stunde mit einem Flutfluss von 200 ml/min durch das Lumen der Faser zirkuliert. Während dieser Zeit wird gleichzeitig ein Filtratfluss von 60 ml/min durch die Faserwand nach außen filtriert. Der Siebkoeffizient für LDL (Low Density Lipoproteine) liegt unter diesen Bedingungen bei mindestens 90 %, typischerweise bei 95-100 %, meistens bei 99 %. Der Siebkoeffizient für LDL bleibt über einen Zeitraum, der mindestens dem Blutbehandlungszeitraum einer gängigen Plasmafiltration entspricht, konstant.

*Gehalt an freiem Polyvinylpyrrolidon*

Der Polyvinylpyrrolidonrückstand aus der erfindungsgemäßen Membran nach Extrakt aus dem Endprodukt betrug < 1 mg. Der letztere Wert ist insbesondere deswegen vorteilhaft, da somit die erfindungsgemäße Hohlfasermembran insbesondere bei Dialysebehandlungen eingesetzt werden, die über sehr lange Zeiträume durchgeführt werden. Insbesondere kann die erfindungsgemäße Membran für Membranpheresebehandlungen eingesetzt werden kann, da hier Grenzwerte von bis zu 5 mg Freisetzung Polyvinylpyrrolidon pro Filter mit 0,6 m$^2$ Membranfläche bestimmt nach der untenstehenden Methode vertretbar sind. Die erfindungsgemäße Membran liegt deutlich unter diesem Grenzwert.

Andere Rückstände als Polyvinylpyrrolidon konnten in dem Extrakt aus dem Filter nicht aufgefunden werden.

Die Extraktion des Polyvinylpyrrolidons erfolgte gemäß der folgenden Vorschrift:

Bei der Extraktion wurden zwei Plasmafilter aus der gleichen Charge verwendet.

Probe Nr. 1 bestand wie Probe 2 aus einem Faserbündel (Gesamt-membranfläche 0,6 m$^2$). Jeder Plasmafilter wurde mit 1000 ml Liter bei 37 Celsius rezirkulierend über 90 Minuten extrahiert.

Der Fluss am blutseitigen Eingang des Plasmafilters betrug 200 ml. 60 ml/min. davon wurden filtriert und 140 ml/min flossen am blutseitigen Ausgang wieder aus dem Filter hinaus.

Sowohl das Wasser am Ausgang der Blutseite als auch das Filtrat wurden in das Lösungsmittelreservoir zurückgeführt.

Bei dem verwendeten Volumen von 1000 ml Wasser entsprachen die Messwerte in mg/l auch den Werten für mg/Filter.

Die Ergebnisse sind in Tabelle 1 dargestellt.

Tabelle 1: Analysenwerte erfindungsgemäßer Hohlfasermembrane

| Parameter | Meßmethode | Einheit | Probennr. 2600-S-0847-1 | Probennr. 2600-S-0847-2 |
|---|---|---|---|---|
| PVP | quantitative IR | mg/Filter | 0,86 | 0,90 |
| GC-flüchtige Substanzen (als Cyclohexanol) | GC-MSD | mg/l | < 0,10 | < 0,10 |

Die Konzentration an Polyvinylpyrrolidon wurde mittels quantitativer IR Spektroskopie bestimmt und wies einen Wert von 0,86 bis 0,90 mg/Filter auf. Zur Auswertung wurde die CO-Schwingungsbande im Wellenzahlenbereich von 1630 - 1735 cm$^{-1}$ herangezogen.

Wie aus Tabelle 1 ersichtlich ist, betragen die Werte für eluierbares PVP in beiden Proben somit weniger als 1 mg/Filter, womit die erfindungsgemäße Hohlfasermembran somit auch strenge Vorschriften im Bezug auf eluierbares PVP erfüllt.

Übliche akzeptable eluierbare PVP Mengen von weniger als 5 mg sind akzeptabel, bevorzugt sind Werte kleiner als 3 mg/Filter, noch mehr bevorzugt kleiner als 2 mg/Filter, ganz besonders bevorzugt von weniger als 1 mg/Filter.

Der gesamte PVP-Gehalt der fertigen Hohlfasermembran liegt bei ca. 3 % (Gewichtsprozent). Die Bestimmung erfolgte dabei beispielsweise über Infrarotspektroskopie oder Pyrolysegaschromatographie mit Stickstoff und Schwefeldetektion.

**Patentansprüche**

1. Hohlfasermembran, bestehend aus zwei koextrudierten Schichten A und B,
   wobei Schicht B eine vliesartige Struktur mit einer Maschenweite von 0,1 bis 10 μm aufweist und
   wobei die Maschenweite in der vliesartigen Struktur der weiteste Abstand zwischen den einzelnen Verzweigungen der das Vlies oder Netz bildenden Struktur ist und
   wobei die Dicke der Stege der Verzweigung 0,1 bis 0,5 μm beträgt, und wobei
   Schicht A eine Porenstruktur aufweist,
   das Material der Schichten A und B eine Mischung aus Polysulfon (PS) und Polyvinylpyrrolidon (PVP) ist,
   wobei die Maschenweite der Maschen in Schicht B größer ist als sämtliche Poren der gesamten Schicht A.

2. Hohlfasermembran nach Anspruch 1, **dadurch gekennzeichnet, dass** der Innendurchmesser der Hohlfasermembran 280 bis 400 μm beträgt.

3. Hohlfasermembran nach Anspruch 2, **dadurch gekennzeichnet, dass** die Gesamtwandstärke der Hohlfasermembran 40 bis 80 μm beträgt.

4. Hohlfasermembran nach einem der Ansprüche 1 bis 3, **dadurch gekennzeichnet, dass** Schicht A aus mindestens 3 Zonen A1, A2, A3 unterschiedlicher Porosität besteht, wobei die Zone A2 zwischen den Zonen A1 und A3 angeordnet ist und Porengrößen von > 200 nm aufweist, die Zone A1 die Oberflächen der Schicht A bildet und Poren mit einer mittleren Porengröße von 0,7-2 μm aufweist und die Zone A3 benachbart zur Schicht B ist und einen Porengrößengradienten zur Schicht B hin aufweist, wobei die Porengröße zur Schicht B hin zunimmt, wobei die Dicke der Zone A1 im Bereich von 9 bis 11 μm, die Dicke der Zone A2 bei 10 μm und die Dicke der Zone A3 bei 30 μm bei einer gesamten Wandstärke von 60 μm liegen.

5. Hohlfasermembran nach Anspruch 3, **dadurch gekennzeichnet, dass** das Verhältnis der Schichtdicken von Schicht A zu Schicht B im Bereich von 4:1 bis 6:1 liegt.

**6.** Hohlfasermembran nach Anspruch 5, **dadurch gekennzeichnet, dass** der eluierbare Anteil an freiem Rest-Polyvinylpyrrolidon in der fertigen Membran weniger als 5 mg/0,6 m$^2$ Membranfläche beträgt, gemessen wie im Beispielabschnitt der Beschreibung beschrieben.

**7.** Hohlfasermembran nach Anspruch 6, **dadurch gekennzeichnet, dass** der LDL (Low Density Lipoprotein) Siebkoeffizient der Hohlfasermembran größer als 0,9 ist, gemessen wie im Beispielabschnitt der Beschreibung beschrieben.

**8.** Verfahren zur Herstellung einer Hohlfasermembran nach einem der vorhergehenden Ansprüche, umfassend die Schritte des

(a) Bereitstellens zweier Spinnmasselösungen A und B, die jeweils eine Mischung aus Polysulfon (PS) und Polyvinylpyrrolidon (PVP) enthalten, wobei die Viskosität der Spinnmasselösung A höher ist als die Viskosität der Spinnmasselösung B
(b) Einstellens der Fällbadtemperatur auf > 70°C
(c) In-Kontakt-bringens der beiden Spinnmasselösungen A und B durch eine Hohlfaserdüse mit einem inneren Fällungsmittel
(d) Fällens der Hohlfasermembran

wobei die Spinngeschwindigkeit 200 bis 400 mm/s beträgt, **dadurch gekennzeichnet, dass**
die Viskosität der Spinnmasselösung A im Bereich von 8000 bis 15000 mPa•s liegt,
wobei die Viskosität der Spinnmasselösung B weniger als 1000 mPa•s beträgt, und
wobei die Fällspalthöhe 5 bis 50 mm beträgt, wobei die Viskositäten wie in der Beschreibung beschrieben gemessen werden.

**9.** Verfahren nach Anspruch 8, **dadurch gekennzeichnet, dass** die Spinnmasselösung A 15 bis 35 % Polysulfon, 4 bis 8 % Polyvinylpyrrolidon und Rest Fällungsmittel enthält.

**10.** Verfahren nach Anspruch 8, **dadurch gekennzeichnet, dass** die Spinnmasselösung B 8 bis 14 % Polysulfon, 3 bis 6 % Polyvinylpyrrolidon und Rest Lösungsmittel enthält.

**11.** Verfahren nach Anspruch 8, **dadurch gekennzeichnet, dass** die Spinnblocktemperatur auf 50 bis 90°C eingestellt wird.

**12.** Verfahren nach Anspruch 11, **dadurch gekennzeichnet, dass** das Fällungsmittel eine Mischung aus Dimethylacetamid und Wasser ist.

**13.** Verwendung einer Hohlfasermembran nach einem der Ansprüche 1 bis 7 für Trennprozesse im Nanofiltrations- und Ultrafiltrationsbereich und für die Hämodialyse, Hämodiafiltration und Hämofiltration.

**Claims**

**1.** Hollow fiber membrane, consisting of two co-extruded layers A and B,
wherein layer B has a non-woven structure with a mesh size of 0.1 to 10 $\mu$m and
wherein the mesh size in the non-woven structure is the greatest distance between the individual branchings of the structure forming the non-woven material or net,
wherein the thickness of the webs of the branching is 0.1 to 0.5 $\mu$m, and wherein layer A has a porous structure,
the material of layers A and B is a mixture of polysulfone (PS) and polyvinylpyrrolidone (PVP),
wherein the mesh size of the meshes in layer B is larger relative to all pores of layer A.

**2.** Hollow fiber membrane according to claim 1, **characterized in that** the internal diameter of the hollow fiber membrane is 280 to 400 $\mu$m.

**3.** Hollow fiber membrane according to claim 2, **characterized in that** the total wall width of the hollow fiber membrane is 40 to 80 $\mu$m.

**4.** Hollow fiber membrane according to any one of claims 1 to 3, characterized in that layer A consists of at least 3 zones A1, A2, A3 of different porosity, wherein zone A2 is arranged between zones A1 and A3 and has pore sizes of > 200 nm, the zone A1 forms the surfaces of layer A and has pores with an average pore size of 0.7-2 $\mu$m, and the zone A3 is adjacent to layer B and has a pore-size gradient towards layer B, wherein the pore size increases towards layer B, wherein the thickness of zone A1 is in the range of 9 to 11 $\mu$m, the thickness of zone A2 is 10 $\mu$m and the thickness of zone A3 is 30 $\mu$m, at a total wall width of 60 $\mu$m.

5. Hollow fiber membrane according to claim 3, **characterized in that** the ration of the layer thicknesses of layer A to layer B is in the range of 4:1 to 6:1.

6. Hollow fiber membrane according to claim 5, **characterized in that** the elutable portion of free residual polyvinylpyrrolidone in the finished membrane is less than 5 mg/0.6 m$^2$ membrane surface, measured as described in the Examples section of the description.

7. Hollow fiber membrane according to claim 6, **characterized in that** the LDL (Low Density Lipoprotein) screening coefficient of the hollow fiber membrane is greater than 0.9, measured as described in the Examples section of the description.

8. Method for the production of a hollow fiber membrane according to any one of the preceding claims, comprising the steps of:

> (a) providing two spinning mass solutions A and B each containing a mixture of polysulfone (PS) and polyvinylpyrrolidone (PVP), wherein the viscosity of the spinning mass solution A is higher than the viscosity of the spinning mass solution B;
> (b) setting the precipitation bath temperature at > 70°C;
> (c) bringing the two spinning mass solutions A and B into contact with an internal precipitant through a hollow fiber spinneret;
> (d) precipitating said hollow fiber membrane

> wherein the spinning speed is 200 to 400 mm/s, **characterized in that**
> the viscosity of spinning mass solution A is in the range of 8000 to 15000 mPa•s,
> wherein the viscosity of spinning mass solution B is less than 1000 mPa•s, and
> wherein the precipitation slit height is 5 to 50 mm, wherein the viscosities are measured as described in the description.

9. Method according to claim 8, **characterized in that** the spinning mass solution A contains 15 to 35% polysulfone, 4 to 8% polyvinylpyrrolidone, the remainder being precipitant.

10. Method according to claim 8, **characterized in that** the spinning mass solution B contains 8 to 14% polysulfone, 3 to 6% polyvinylpyrrolidone, the remainder being solvent.

11. Method according to claim 8, **characterized in that** the spinning block temperature is set to 50 to 90°C.

12. Method according to claim 11, **characterized in that** the precipitant is a mixture of dimethylacetamide and water.

13. Use of a hollow fiber membrane according to any one of claims 1 to 7 for separation processes in the nanofiltration and ultrafiltration ranges and for haemodialysis, haemodiafiltration and haemofiltration.

**Revendications**

1. Membrane à fibres creuses constituée de deux couches co-extrudées A et B,
   dans laquelle la couche B a une structure non tissée avec un maillage de 0,1 à 10 $\mu$m et
   dans laquelle le maillage dans la structure non tissée est la plus grande distance entre les ramifications individuelles de la structure formant le matériau non tissé ou le filet,
   dans laquelle l'épaisseur des nappes de ramification est comprise entre 0,1 et 0,5 $\mu$m et dans laquelle la couche A a une structure poreuse,
   le matériau des couches A et B est un mélange de polysulfone (PS) et de polyvinylpyrrolidone (PVP),
   dans lequel le maillage de la couche B est plus grande par rapport à tous les pores de la couche A.

2. Membrane à fibres creuses selon la revendication 1, **caractérisée en ce que** le diamètre interne de la membrane à fibres creuses est compris entre 280 et 400 μm.

3. Membrane à fibres creuses selon la revendication 2, **caractérisée en ce que** la largeur de paroi totale de la membrane à fibres creuses est comprise entre 40 et 80 μm.

4. Membrane à fibres creuses selon l'une quelconque des revendications 1 à 3, **caractérisée en ce que** la couche A est constituée d'au moins 3 zones A1, A2, A3 de porosités différentes, la zone A2 étant disposée entre les zones A1 et A3 et ayant des tailles de pores > 200 nm, la zone A1 formant les surfaces de la couche A et ayant des pores d'une taille moyenne de pores de 0,7 à 2 μm, et la zone A3 étant adjacente à la couche B et ayant un gradient de taille de pores vers la couche B, dans lequel la taille des pores augmente en direction de la couche B, l'épaisseur de la zone A1 étant comprise entre 9 et 11 μm, l'épaisseur de la zone A2 étant 10 μm et l'épaisseur de la zone A3 30 μm, pour une largeur totale de paroi de 60 μm.

5. Membrane à fibres creuses selon la revendication 3, **caractérisée en ce que** le rapport des épaisseurs de couche de la couche A à la couche B est compris entre 4: 1 et 6: 1.

6. Membrane à fibres creuses selon la revendication 5, **caractérisée en ce que** la partie éligible de polyvinylpyrrolidone résiduelle libre dans la membrane finie est inférieure à 5 mg/0,6 m$^2$ de surface de membrane, mesurée comme décrit dans la section Exemples de la description.

7. Membrane à fibres creuses selon la revendication 6, **caractérisée en ce que** le coefficient de criblage des LDL (lipoprotéines de basse densité) de la membrane à fibres creuses est supérieur à 0,9, mesuré comme décrit dans la section Exemples de la description.

8. Procédé de fabrication d'une membrane à fibres creuses selon l'une quelconque des revendications précédentes, comprenant les étapes de:

   (a) fournir deux solutions de masses de filage A et B contenant chacune un mélange de polysulfone (PS) et de polyvinylpyrrolidone (PVP), la viscosité de la solution de masse de filage A étant supérieure à la viscosité de la solution de masse de filage B;
   (b) régler la température du bain de précipitation à > 70 °C;
   (c) mettre les deux solutions de masses de filage A et B en contact avec un précipitant interne à travers une filière à fibres creuses;
   (d) précipiter ladite membrane à fibres creuses

   dans lequel la vitesse de filage est comprise entre 200 et 400 mm/s, **caractérisée en ce que**
   la viscosité de la solution de masse de filage A est comprise entre 8 000 et 15 000 mPa• s,
   dans lequel la viscosité de la solution de masse de filage B est inférieure à 1000 mPa•s, et
   dans lequel la hauteur de la fente de précipitation est comprise entre 5 et 50 mm, les viscosités étant mesurées comme décrit dans la description.

9. Procédé selon la revendication 8, **caractérisé en ce que** la solution de masse de filature A contient de 15 à 35% de polysulfone, de 4 à 8% de polyvinylpyrrolidone, le reste étant un précipitant.

10. Procédé selon la revendication 8, **caractérisé en ce que** la solution de masse filée B contient 8 à 14% de polysulfone, 3 à 6% de polyvinylpyrrolidone, le reste étant un solvant.

11. Procédé selon la revendication 8, **caractérisé en ce que** la température du bloc de filage est réglée entre 50 et 90 °C.

12. Procédé selon la revendication 11, **caractérisé en ce que** le précipitant est un mélange de diméthylacétamide et d'eau.

13. Utilisation d'une membrane à fibres creuses selon l'une quelconque des revendications 1 à 7 pour des procédés de séparation dans les domaines de la nanofiltration et de l'ultrafiltration et pour l'hémodialyse, l'hémodiafiltration et l'hémofiltration.

Abbildung 1

Abbildung 2

Abbildung 3

Abbildung 4

Abbildung 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9637282 A
- EP 1547628 A1
- US 6565782 B
- US 5141642 A
- US 5472607 A
- US 2003232184 A1
- US 5145583 A
- US 2006108288 A1
- US 5863645 A
- US 7172075 B1
- US 5298206 A
- JP 2001038171 A
- DE 10211051 A1
- DE 10211051


**In der Beschreibung aufgeführte Nicht-Patentliteratur**

- **HÖRL et al.** Replacement of Renal Function by Dialysis. Kluwer, 2004, 709-724
- **M. MULDER.** Basic Principles of Membrane Technology second. Kluwer, 1996, 71-91